Europäisches Patentamt

(19) European Patent Office

Office européen des brevets

(11) Publication number: **0 027 429**
**B1**

(12) **EUROPEAN PATENT SPECIFICATION**

(45) Date of publication of patent specification: **20.02.85**

(21) Application number: **80810238.8**

(22) Date of filing: **28.07.80**

(51) Int. Cl.⁴: **A 61 K 35/22, A 61 K 35/24,**
**A 61 K 35/14, A 61 K 35/12**

(54) **A process for preparing tumor antidotes (oncoclasines) from the excrements of goats.**

(30) Priority: **11.10.79 CH 9195/79**

(43) Date of publication of application:
**22.04.81 Bulletin 81/16**

(45) Publication of the grant of the patent:
**20.02.85 Bulletin 85/08**

(84) Designated Contracting States:
**AT BE DE FR GB LU NL SE**

(56) References cited:
**FR-A-2 161 779**
**GB-A-1 326 928**

**BIOLOGICAL ABSTRACTS, vol. 65, 15-04-1978,**
**abstract 45712, W.L. McGUIRE et al.: "Progress**
**in Cancer Research and Therapy", vol. 4,**
**Progesterone receptors in normal and**
**neoplastic tissues**

(73) Proprietor: **Bonifacio Gaetana**
**Via Q. Sella no.5**
**Agropoli (Salerno) (IT)**

(73) Proprietor: **Bonifacio Calogero**
**Via Q. Sella no.5**
**Agropoli (Salerno) (IT)**

(73) Proprietor: **Bonifacio Angelo**
**Via Q. Sella no.5**
**Agropoli (Salerno) (IT)**

(73) Proprietor: **Bonifacio Guiseppe**
**Via Q. Sella no.5**
**Agropoli (Salerno) (IT)**

(73) Proprietor: **Bonifacio Leonardo**
**Via Q. Sella no.5**
**Agropoli (Salerno) (IT)**

(72) Inventor: **Bonifacio, Liborio**
**deceased**
**Agropoli (Salerno) (IT)**

(74) Representative: **Baggiolini, Raimondo**
**Racheli & Fiammenghi Via San Gottardo 15**
**CH-6900 Lugano (CH)**

Courier Press, Leamington Spa, England.

## Description

Processes to prepare tumor antidotes from the body of animals are known, see for example:

Volume 65 Biological Abstracts, 15.04.1978, abstract 45712, W. L. McGuire et al.: "Progress in Cancer Research and Therapy";

French Patent No. 2,161,779 (Taiho Pharmaceutical Company Limited); and

British Patent No. 1,326,928 (Institut Pasteur).

The inventor proceeds from the observation that goats never have tumors or cancerous growths.

After long study and research, excrements (feces and urine) have been extracted from the male goat and female goat, in the form of two varities of tumor antidotes (oncoclasine) which have been respectively designated as "M" and "F", the first used for the cure of carcinomas and all tumors of the epithetial tissue, except for carcinoma of the prostate, the second for the cure of sarcomas and tumors of the connective tissue, including leukemia and carcinomas of the prostate.

The process of extraction consists of mixing the feces and the urine of the same animal with a certain quantity of water which is preferably twice-distilled, allowing the mixture to stand filtering the mixture first through a normal paper filter and then through a sterilizing filter, for example of the "millipore" type.

Following are the particular details of the process of extraction of the "M" and "F" tumor antidotes from the excrement, that is to say from the feces and the urine, of the same animal respectively of male or female sex.

The "M" tumor antidote which serves for the cure of carcinomas and generally of all tumors of the epithelial tissue, except for carcinomas of the prostate, is obtained in the following manner:

— the feces the male goat are mixed with some parts of urine of the same animal, adding approximately one third part of twice-distilled water;

— the mixture is allowed to stand, in other words to amalgamate, for approximately 48 hours;

— it is filtered, first through normal filter paper and then through a sterilizing filter, which are called "millipores", thereby obtaining a dark straw-yellow liquid substance which serves for the cure of the tumors.

The process for the extraction of the tumor antidote "F" which serves for the cure of sarcomas and tumors of the connective tissue, including leukemia and carcinomas of the prostate, include the following operations:

— the feces of the female goat are mixed with urine of the same animal, adding approximately one third part of twice-distilled water;

— the mixture is allowed to stand, in other words to amalgamate, for approximately 48 hours;

— it is filtered, first through a normal filter paper and then through a sterilizing filter, which are called "millipores", thus obtaining a liquid substance of dark straw-yellow color, which serves for the cure of the tumors specified above.

## Claims

1. Process for preparing antidotes (oncoclasines) for the cure respectively of the following two classes of tumors:

a) carcinomas and generally all tumors of the epithelial tissue, except for prostate carcinomas;

b) sarcomas and tumors of the connective tissue, including leukemia and prostate carcinomas;

characterized by the following operations:

— for the cure of the tumors of class a), the extraction of an antidote (M) from the feces and urine of the *male goat*, by mixing the feces and urine with water allowing the mixture to stand and filtering it through normal filter paper and then through sterilizing filter;

— for the cure of the tumors of class b), the extraction of an antidote (F) from the feces and urine of the *female goat*, by mixing feces and urine with water allowing the mixture to stand and filtering it through normal filter paper and then through sterilizing filter.

2. Process for the extraction of the tumor antidote "M" as in claim 1, characterized by the following operations:

— the feces of the *male goat* are mixed with urine of the same animal, adding approximately 1/3 part of twice-distilled water;

— the mixture is allowed to stand for approximately 48 hours;

— it is filtered, first through a normal filter paper then through a sterilizing filter ("millipores"), thus obtaining a liquid substance of dark straw-yellow color, which cures the tumors specified in class a).

3. Process for the extraction of the tumor antidote "F" as in claim 1, characterized by the following operations:

— the feces of the *female goat* are mixed with urine of the same animal, adding approximately 1/3 part of twice-distilled water;

— the mixture is allowed to stand for approximately 48 hours;

— it is filtered, first through a normal filter paper and then through a sterilizing filter

(millipores), thus obtaining a liquid substance of dark straw-yellow color, which cures the tumors specified in class b).

4. Antidotes M and F obtainable by the process according to claim 1.

**Patentansprüche**

1. Verfahren zum Herstellen von Tumorgegenmitteln (Oncoclasinen) zum Behandeln der folgenden zwei Klassen von Tumoren, u.zw.
   a) Carcinomen und allgemein allen Tumoren des Epithelgewebes mit Ausnahme von Prostatacarcinomen und
   b) Sarcomen und Tomoren des Bindegewebes einschließlich Leukämie und Prostatacarcinomen,
dadurch gekennzeichnet,

— daß für die Behandlung von Tumoren der Klasse a), ein Tumorgegenmittel (M) aus den Fäzes und dem Urin der männlichen Ziege durch Vermischen der Fäzes und Urin mit Wasser extrahiert wird und das Gemisch stehengelassen und durch ein normales Papierfilter und dann durch ein sterilisierendes Filter filtriert wird oder
— daß für die Behandlung von Tumoren der Klasse b) ein Tumorgegenmittel (F) aus den Fäzes und Urin der weiblichen Ziege durch Vermischen von Fäzes und Urin mit Wasser extrahiert wird und das Gemisch stehengelassen und durch ein normales Papierfilter und dann durch ein sterilisierendes Filter filtriert wird.

2. Verfahren zum Extrahieren des Tumorgegenmittels (M) nach Anspruch 1, dadurch gekennzeichnet, daß

— die Fäzes der männlichen Ziege mit Urin des gleichen Tieres vermischt werden und etwa 1/3 Teil zweifach destillierten Wasser zugegeben wird und
— das Gemisch etwa 48 h stehengelassen und
— dann zunächst durch ein normales Papierfilter und anschließend durch ein sterilisierendes Filter ("Millipores") filtriert wird, womit eine flüssige Substanz dunkler strohgelber Farbe erhalten wird, welche Tumore der in Klasse a) angegebenen Art heilt.

3. Verfahren zum Extrahieren des Tumorgegenmittels "F" gemäß Anspruch 1, dadurch gekennzeichnet,

— daß die Fäzes der weiblichen Ziege mit Urin des gleichen Tieres vermischt werden und etwa 1/3 Teil zweifach destillierten Wassers zugesetzt wird und
— daß das Gemisch während etwa 48 h stehengelassen und
— dann zunächst durch ein normales Papierfilter und anschließend durch ein sterilisierendes Filter ("Millipores") filtriert wird, womit eine flüssige Substanz von dunkler strohgelber Farbe erhalten wird, welche die Tumore der in Klasse b) angegebenen Art heilt.

4. Tumorgegenmittel M und F wie sie nach dem Verfahren gemäß Anspruch 1 erhältlich sind.

**Revendications**

1. Procédé pour prépare des antidotes (oncoclasines) pour le traitement respectivement des deux classes suivantes de tumeurs:
   a) carcinomes et de façon générale toutes tumeurs du tissue épithélial, à l'exception des carcinomes de la prostate;
   b) sarcomes et tumeurs du tissue conjonctif, comprenant la leucémie et les carcinomes de la prostate;
caractérisé par les opérations suivantes:

— pour le traitement des tumeurs de classe a), extraction d'un antidote (M) des fèces et de l'urine du bouc par mélange des fèces et de l'urine avec de l'eau en laissant reposer le mélange et en le filtrant à travers du papier filtre normal, puis à travers un filtre stérilisant;
— pour le traitement des tumeurs de la classe b), extraction d'un antidote (F) des fèces et de l'urine de la chèvre femelle par mélange des fèces et de l'urine avec de l'eau en laissant reposer le mélange et en le filtrant à travers du papier filtre normal, puis à travers un filtre stérilisant.

2. Procédé pour l'extraction de l'antidote de tumeurs "M" selon la revendication 1, caractérisé par les opérations suivantes:

— on mélange les fèces du bouc avec l'urine du même animal en ajoutant approximativement 1/3 d'eau, deux fois distillée;
— on laisse reposer le mélange pendant approximativement 48 heures;
— on le filtre, d'abord à travers du papier filtre normal, puis à travers un filtre stérilisant ("millipores") en obtenant ainsi une substance liquide d'une coloration jaune paille foncée qui traite les tumeurs spécifiées de classe a).

3. Procédé pour l'extraction de l'antidote de tumeurs "F" selon la revendication 1, caractérisé par les opérations suivantes:

— on mélange les fèces de la chèvre femelle avec l'urine du même animal en ajoutant approximativement 1/3 d'eau, deux fois distillée;

— on laisse reposer le mélange pendant approximativement 48 heures;
— on le filtre d'abord à travers du papier filtre normal, puis à travers un filtre stérilisant ("millipores") en obtenant ainsi une substance liquide d'une coloration jaune paille foncée qui traite les tumeurs spécifiées de classe b).

4. Antidotes M et F obtenus par le procédé selon la revendication 1.